# NEUE EUROPÄISCHE PATENTSCHRIFT

(11) **EP 1 613 576 B2**
(45) Veröffentlichungstag und Bekanntmachung des Hinweises auf die Entscheidung über den Einspruch: **16.10.2013**
(45) Hinweis auf die Patenterteilung: 26.05.2010
(21) Anmeldenummer: 04722176.7
(22) Anmeldetag: 20.03.2004
(51) Int. Cl.: C07C 29/149, B01J 23/83, B01J 21/04

(54) **VERFAHREN ZUR HYDRIERUNG VON CARBONYLVERBINDUNGEN**
METHOD FOR THE HYDROGENATION OF CARBONYL COMPOUNDS
PROCEDE POUR HYDROGENER DES COMPOSES CARBONYLE

(30) Priorität: 27.03.2003 DE 10313702
(43) Veröffentlichungstag der Anmeldung: 11.01.2006
(73) Patentinhaber: BASF SE, 67056 Ludwigshafen (DE)
(72) Erfinder: HUBER-DIRR, Sylvia, 64673 Zwingenberg (DE); HESSE, Michael, 67549 Worms (DE); HAUNERT, Andrea, 68163 Mannheim (DE); JUNICKE, Henrik, 68165 Mannheim (DE)
(86) Internationale Anmeldenummer: PCT/EP2004/002929
(87) Internationale Veröffentlichungsnummer: WO 2004/085356

(56) Entgegenhaltungen:
- WO-A1-01/17934
- WO-A1-02/47818
- WO-A1-96/14280
- DE-A- 19 505 347
- DE-A- 19 607 955
- US-A- 4 287 099
- US-A- 4 738 946
- US-A- 5 475 159
- US-A- 5 990 040

## Beschreibung

Die vorliegende Erfindung betrifft ein Verfahren zur Hydrierung von organischen Verbindungen, die mindestens eine Carbonylgruppe aufweisen, unter Verwendung eines Formkörpers der sich unter anderem dadurch auszeichnet, dass dieser Kupferoxid, Aluminiumoxid und Lanthanoxid, enthält und dass durch die Zugabe von Lanthanoxid, ein Formkörper mit hoher Selektivität und gleichzeitig hoher Stabilität entsteht. Bei seiner Herstellung wird zusätzlich metallisches Kupferpulver oder Kupferblättchen, zugegeben.

Die katalytische Hydrierung von Carbonylverbindungen wie beispielsweise Carbonsäuren oder Carbonsäureestem nimmt in den Produktionssträngen der chemischen Grundstoffindustrie eine bedeutende Stellung ein.

Die katalytische Hydrierung von Carbonylverbindungen wie z.B. Carbonsäureestem wird in technischen Verfahren fast ausschließlich in Festbettreaktoren durchgeführt. Als Festbettkatalysatoren werden, neben Katalysatoren vom Raney-Typ, vor allem geträgerte Katalysatoren, beispielsweise Kupfer-, Nickel- oder Edelmetall-Katalysatoren verwendet.

Die US 3,923,694 beschreibt beispielsweise einen Katalysator vom Typ Kupferoxid /Zinkoxid / Aluminiumoxid. Der Nachteil dieses Katalysators besteht darin, dass er während der Reaktion mechanisch nicht ausreichend stabil ist und daher relativ schnell zerfällt. Daraus resultiert ein Aktivitätsverlust und ein Aufbau von Differenzdruck über den Reaktor durch die zerfallenden Katalysator-Formkörper. In der Folge muss die Anlage vorzeitig abgestellt werden.

Die DE 198 09 418.3 beschreibt ein Verfahren zur katalytischen Hydrierung einer Carbonylverbindung in Gegenwart eines Katalysators, der einen Träger, der vornehmlich Titandioxid enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfasst, wobei die Kupferoberfläche maximal 10 m²/g beträgt. Bevorzugte Trägermaterialien sind Mischungen aus Titandioxid mit Aluminiumoxid oder Zirkonoxid oder Aluminiumoxid und Zirkonoxid.

In einer bevorzugten Ausführungsform wird das Katalysatormaterial unter Zusatz von metallischem Kupferpulver oder Kupferblättchen verformt.

Die DE-A 195 05 347 beschreibt ganz allgemein ein Verfahren von Katalysatortabletten mit hoher mechanischer Festigkeit, wobei dem zu tablettirrenden Material ein Metallpulver oder ein Pulver einer Metall-Legierung zugegeben wird. Unter anderem wird als Metallpulver Aluminiumpulver oder Kupferpulver oder Kupferblättchen zugegeben. Bei der Zugabe von Aluminiumpulver wird bei einem Kupferoxid / Zinkoxid / Aluminiumoxid-Katalysator allerdings ein Formkörper erhalten, der eine schlechtere Seitendruckfestigkeit aufweist als ein Formkörper, der ohne Zusatz von Aluminiumpulver hergestellt wurde, und der erfindungsgemäße Formkörper zeigte bei seiner Verwendung als Katalysator eine schlechtere Konvertierungsaktivität als Katalysatoren, die ohne Zusatz von Aluminiumpulver hergestellt wurden. Ebenfalls offenbart ist dort ein Hydrierkatalysator aus NiO, ZrO2, MoO3 und CuO, dem bei der Herstellung unter anderem Cu-Pulver zugemischt wurde. Über die Selektivität oder die Aktivität sind in dieser Schrift jedoch keine Angaben gemacht.

Die DE 256 515 beschreibt ein Verfahren zur Herstellung von Alkoholen aus Synthesegas, wobei Katalysatoren auf der Basis von Cu / Al / Zn eingesetzt werden, die durch gemeinsame Vermahlung und Verpillung mit metallischem Kupferpulver oder Kupferblättchen gewonnen werden. Das Hauptaugenmerk liegt bei dem beschriebenen Verfahren auf der Herstellung von Gemischen aus C1- bis C5-Alkoholen, wobei eine Verfahrensführung gewählt wird, in dem der Reaktionsreaktor im oberen Schichtdrittel einen Katalysator enthält, der einen höheren Anteil an Kupferpulver oder Kupferblättchen aufweist, und im unteren Drittel einen Katalysator enthält, der einen geringeren Anteil an Kupferpulver oder Kupferblättchen aufweist.

Eine Aufgabe der vorliegenden Erfindung war es, Verfahren bereitzustellen, die die Nachteile des Standes der Technik nicht aufweisen und Verfahren zur katalytischen Hydrierung von Carbonylverbindungen bereitzustellen, wobei die Katalysatoren sowohl hohe mechanische Stabilität als auch hohe Hydrieraktivität und Selektivität aufweisen.

Es wurde gefunden, dass durch die simultane Fällung von Kupfer-, Aluminium- und Lanthanverbindung, und durch die anschließende Trocknung, Calcinierung, Tablettierung und durch die Zugabe von metallischem Kupferpulver, oder Kupferblättchen, ein Katalysator erhalten wird, der durch die Zugabe einer Lanthanverbindung, sowohl zu hohen Aktivitäten und Selektivitäten sowie zu einer hohen Stabilität des Formkörpers, der als Katalysator eingesetzt wird, führt.

Demgemäss betrifft die vorliegende Erfindung ein Verfahren zur Hydrierung einer mindestens eine Carbonylgruppe aufweisenden organischen Verbindung, bei dem die organische Verbindung in Anwesenheit von Wasserstoff mit einem Formkörper in Kontakt gebracht wird, der herstellbar ist gemäß einem Verfahren, in dem
(i) ein oxidisches Material, umfassend Kupferoxid, Aluminiumoxid und Lanthanoxid, bereitgestellt wird,
(ii) dem oxidischen Material pulverförmiges metallisches Kupfer oder, Kupferblättchen, zugegeben werden, und
(iii) das aus (ii) resultierende Gemisch zu einem Formkörper verformt wird.

Der in dem erfindungsgemäßen Verfahren verwendete Katalysator zeichnet sich dadurch aus, dass die Aktivkomponente Kupfer, die Komponente Aluminium und die Komponente Lanthanoxid bevorzugt mit einer Sodalösung simultan oder nacheinander gefällt werden, im Anschluss getrocknet, calciniert , tablettiert und nochmals calciniert wird.

Insbesondere kommt folgende Fällungsmethode in Betracht:
A) Eine Kupfersalzlösung, eine Aluminiumsalzlösung und eine Lanthansalzlösung oder eine Lösung, enthaltend Kupfer-, Aluminium- und Lanthansalz, wird parallel oder nacheinander mit einer Sodalösung gefällt. Das gefällte Material im Anschluss getrocknet und ggf. calciniert.
B) Fällung einer Kupfersalzlösung und einer Lanthansalzlösung oder einer Lösung, enthaltend Kupfersalz und, Lathansalz auf einen vorgefertigten Aluminiumoxidträger. Dieser liegt in einer besonders bevorzugten Ausführungsform als Pulver in einer wässrigen Suspension vor. Das Trägermaterial kann aber auch als Kugeln, Stränge, Splitt oder Tabletten vorliegen.
B1) In einer Ausführungsform (I) wird eine Kupfersalzlösung und eine Lanthansalzlösung oder ein Lösung, enthaltend Kupfersalz und Lanthansalz bevorzugt mit Sodalösung, gefällt. Als Vorlage wird eine wässrige Suspension des Trägermaterials Aluminiumoxid verwendet.

Ausgefällte Niederschläge, die aus A) oder B) resultieren, werden in üblicher Weise filtriert und vorzugsweise alkalifrei gewaschen, wie dies beispielsweise in der DE 198 09 418.3 beschrieben ist.

Sowohl die Endprodukte aus A) als auch die aus B) werden bei Temperaturen von 50 bis 150°C, vorzugsweise bei 120°C getrocknet und im Anschluß ggf. vorzugsweise 2 Stunden bei im allgemeinen 200 bis 600°C, insbesondere bei 300 bis 500°C calciniert.

Als Ausgangssubstanzen für A) und/oder B) können prinzipiell alle in den bei der Aufbringung verwendeten Lösungsmitteln löslichen Cu(I) und/oder Cu(II)-Salze, wie beispielsweise Nitrate, Carbonate, Acetate, Oxalate oder Ammonium-Komplexe, analoge Aluminiumsalze und Salze des Lanthans, verwendet werden. Besonders bevorzugt für Verfahren gemäß A) und B) wird Kupfernitrat eingesetzt.

In dem erfindungsgemäßen Verfahren wird das oben beschriebene getrocknete und gegebenenfalls calcinierte Pulver bevorzugt zu Tabletten, Ringen, Ringtabletten, Extrudaten, Wabenkörpern oder ähnlichen Formkörpern verarbeitet. Hierfür sind sämtliche aus dem Stand der Technik geeigneten Verfahren denkbar.

Die Zusammensetzung des oxidischen Material ist im allgemeinen so beschaffen, dass der Anteil an Kupferoxid im Bereich von 40 bis 90 Gew.-%, der Anteil an Oxiden des Lanthans, Wolframs, Molybdäns, Titans oder Zirkoniums im Bereich von 0 bis 50 Gew.-% und der Anteil an Aluminiumoxid im Bereich bis zu 50 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

Das erfindungsgemäße Verfahren zeichnet sich dadurch aus, dass durch die Zugabe von Lanthan, bei der Fällung zu einer hohen Stabilität des Formkörpers, der als Katalysator eingesetzt wird, führt.

Ein Beispel für Zement ist ein Tonerdezement. Tonerdezement besteht im wesentlichen aus Aluminiumoxid und Calciumoxid, beispielsweise aus ungefähr 75 bis 85 Gew.-% Aluminiumoxid und ungefähr 15 bis 25 Gew.-% Calciumoxid. Ferner kann ein Zement auf Basis Magnesiumoxid/Aluminiumoxid, Calciumoxid/Siliciumoxid und Calciumoxid/Aluminiumoxid/Eisenoxid verwendet werden.

Insbesondere kann das oxidische Material in einem Anteil von höchstens 10 Gew.-%, bevorzugt höchstens 5 Gew.-%, bezogen auf das Gesamtgewicht des oxidischen Materials, mindestens eine weitere Komponente aufweisen, die ausgewählt wird aus der Gruppe bestehend aus den Elementen Re, Fe, Ru, Co, Rh, Ir, Ni, Pd und Pt.

In einer weiter bevorzugten Ausführungsform des erfindungsgemäßen Verfahrens wird dem oxidischen Material vor dem Verformen zum Formkörper zusätzlich zu dem Kupferpulver, oder Kupferblättchen Graphit zugesetzt. Vorzugsweise wird soviel Graphit zugegeben, dass die Verformung zu einem Formkörper besser durchgeführt werden kann. In einer bevorzugten Ausführungsform werden 0,5 bis 5 Gew.-% Graphit, bezogen auf das Gesamtgewicht des oxidischen Materials, zugegeben. Dabei ist es gleichgültig, ob Graphit dem oxidischen Material vor oder nach oder gleichzeitig mit dem Kupferpulver oder den Kupferblättchen zugesetzt wird.

Demgemäss betrifft die vorliegende Erfindung auch ein Verfahren, wie oben beschrieben, das dadurch gekennzeichnet, ist, dass dem oxidischen Material oder dem aus (ii) resultierendem Gemisch Graphit in einem Anteil im Bereich von 0,5 bis 5 Gew.-%, bezogen auf das Gesamtgewicht des oxidischen Materials, zugegeben wird.

Nach Zugabe des Kupferpulvers, oder der Kupferblättchen und gegebenenfalls Graphit zu dem oxidischen Material wird der im Anschluß an die Verformung erhaltene Formkörper gegebenenfalls mindestens einmal calciniert über eine Zeit von im allgemeinen 0,5 bis 10 h, bevorzugt 0,5 bis 2 Stunden. Die Temperatur bei diesem mindestens einen Calcinierschritt liegt im allgemeinen im Bereich von 200 bis 600°C, bevorzugt im Bereich von 250 bis 500°C und besonders bevorzugt im Bereich von 270 bis 400°C.

Bei Einsatz als Katalysator in der oxidischen Form wird der Formkörper vor Beschickung mit der Hydrierlösung mit reduzierenden Gasen, beispielsweise Wasserstoff, vorzugsweise Wasserstoff-Inertgasgemischen, insbesondere Wasserstoff/Stickstoffgemischen bei Temperaturen im Bereich von 100 bis 500°C, bevorzugt im Bereich von 150 bis 350°C und insbesondere im Bereich von 180 bis 200°C vorreduziert. Bevorzugt wird dabei ein Gemisch mit einem Wasserstoffanteil im Bereich von 1 bis 100 Vol.-%, besonders bevorzugt im Bereich von 1 bis 50 Vol.-% verwendet.

In einer bevorzugten Ausführungsform wird der Formkörper vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Reaktor vorab im Reduktionsofen aktiviert worden, wird er in den Reaktor eingebaut und direkt unter Wasserstoffdruck mit der Hydrierlösung beschickt.

Bevorzugtes Einsatzgebiet der Formkörper ist die Hydrierung von Carbonylgruppen aufweisenden organischen Verbindungen im Festbett. Andere Ausführungsformen wie beispielsweise die Wirbelreaktion mit in auf- und abwirbelnder Bewegung befindlichem Katalysatormaterial ist jedoch ebenfalls möglich. Die Hydrierung kann in der Gasphase oder in der Flüssigphase durchgeführt werden. Vorzugsweise wird die Hydrierung in flüssiger Phase durchgeführt, beispielsweise in Riesel- oder Sumpffahrweise.

Bei Arbeiten in Rieselfahrweise lässt man das flüssige, die zu hydrierende Carbonylverbindung enthaltende Edukt in dem Reaktor, der unter Wasserstoffdruck steht, über das in diesem angeordnete Katalysatorbett rieseln, wobei sich auf dem Katalysator ein dünner Flüssigkeitsfilm ausbildet. Dagegen wird beim Arbeiten in Sumpffahrweise Wasserstoffgas in den mit der flüssigen Reaktionsmischung gefluteten Reaktor eingeleitet, wobei der Wasserstoff das Katalysatorbett in aufsteigenden Gasperlen passiert.

In einer Ausführungsform wird die zu hydrierende Lösung im geraden Durchgang über die Katalysatorschüttung gepumpt. In einer anderen Ausführungsform des erfindungsgemäßen Verfahrens wird ein Teil des Produkts nach Durchgang durch den Reaktor als Produktstrom kontinuierlich abgezogen und ggf. durch einen zweiten Reaktor, wie oben definiert, geleitet. Der andere Teil des Produkts wird zusammen mit frischem, die Carbonylverbindung enthaltendem Edukt dem Reaktor erneut zugeführt. Diese Verfahrensweise wird im folgenden als Kreislauffahrweise bezeichnet.

Wird als Ausführungsform des erfindungsgemäßen Verfahrens die Rieselfahrweise gewählt, ist hierbei die Kreislauffahrweise bevorzugt. Weiter bevorzugt wird in Kreislauffahrweise unter Verwendung eines Haupt- und Nachreaktors gearbeitet.

Das erfindungsgemäße Verfahren eignet sich zur Hydrierung von Carbonylverbindungen wie z.B. Aldehyden und Ketonen, Carbonsäuren, Carbonsäureestern oder Carbonsäureanhydriden zu den entsprechenden Alkoholen, wobei aliphatische und cycloaliphatische gesättigte und ungesättigte Carbonylverbindungen bervorzugt sind. Bei aromatischen Carbonylverbindungen kann es zur Bildung unerwünschter Nebenprodukte durch Hydrierung des aromatischen Kerns kommen. Die Carbonylverbindungen können weitere funktionelle Gruppen wie Hydroxy- oder Aminogruppen tragen. Ungesättigte Carbonylverbindungen werden in der Regel zu den entsprechenden gesättigten Alkoholen hydriert. Der Begriff "Carbonylverbindungen", wie er im Rahmen der Erfindung verwendet wird, umfaßt alle Verbindungen, die eine C=O-Gruppe aufweisen, einschließlich Carbonsäuren und deren Derivaten. Selbstverständlich können auch Gemische aus zwei oder mehr als zwei Carbonylverbindungen gemeinsam hydriert werden. Ferner kann auch die einzelne, zu hydrierende Carbonylverbindung mehr als eine Carbonylgruppe enthalten.

Bevorzugt wird das erfindungsgemäße Verfahren zur Hydrierung aliphatischer Aldehyde, Hydroxyaldehyde, Ketone, Säuren, Ester, Anhydride, Lactone und Zucker eingesetzt.

Bevorzugte aliphatische Aldehyde sind verzweigte und unverzweigte gesättigte und/oder ungesättigte aliphatische C₂-C₃₀-Aldehyde, wie sie beispielsweise durch Oxosynthese aus linearen oder verzweigten Olefinen mit interner oder terminaler Doppelbindung erhältlich sind. Ferner können auch oligomere Verbindungen, die auch mehr als 30 Carbonylgruppen enthalten, hydriert werden.

### Als Beispiel für aliphatische Aldehyde sind zu nennen:

Formaldehyd, Propionaldehyd, n-Butyraldehyd, iso-Butyraldehyd, Valeraldehyd, 2-Methylbutyraldehyd, 3-Methylbutyraldehyd (Isovaleraldehyd), 2,2-Dimethylpropionaldehyd (Pivalinaldehyd), Capronaldehyd, 2-Methylvaleraldehyd, 3-Methylvaleraldehyd, 4-Methylvaleraldehyd, 2-Ethylbutyraldehyd, 2,2-Dimethylbutyraldehyd, 3,3-Dimethylbutyraldehyd, Caprylaldehyd, Caprinaldehyd, Glutardialdehyd.

Neben den genannten kurzkettigen Aldehyden sind insbesondere auch langkettige aliphatische Aldehyde geeignet, wie sie beispielsweise durch Oxosynthese aus linearen α-Olefinen erhalten werden können.

Besonders bevorzugt sind Enalisierungsprodukte, wie z.B. 2-Ethylhexenal, 2-Methylpentenal, 2,4-Diethyloctenal oder 2,4-Dimethylheptenal.

Bevorzugte Hydroxyaldehyde sind C₃-C₁₂-Hydroxyaldehyde, wie sie beispielsweise durch Aldolreaktion aus aliphatischen und cycloaliphatischen Aldehyden und Ketonen mit sich selbst oder Formaldehyd zugänglich sind. Beispiele sind 3-Hydroxypropanal, Dimethylolethanal, Trimethylolethanal (Pentaerythrital), 3-Hydroxybutanal (Acetaldol), 3-Hydrox-y-2-ethylhexanal (Butylaldol), 3-Hydroxy-2-methylpentanal (Propienaldol), 2-Methylolpropanal, 2,2-Dimethylolpropanal, 3-Hydroxy-2-methylbutanal, 3-Hydroxypentanal, 2-Methylolbutanal, 2,2-Dimethytolbutanal, Hydroxypivalinaldehyd. Besonders bevorzugt sind Hydroxypivalinaldehyd (HPA) und Dimethylolbutanal (DMB).

Bevorzugte Ketone sind Aceton, Butanon, 2-Pentanon, 3-Pentanon, 2-Hexanon, 3-Hexanon, Cyclohexanon, Isophoron, Methylisobutylketon, Mesityloxid, Acetophenon, Propiophenon, Benzophenon, Benzalaceton, Dibenzalaceton, Benzalacetophenon, 2,3-Butandion, 2,4-Pentandion, 2,5-Hexandion und Methylvinylketon.

Darüber hinaus können Carbonsäuren und Derivate davon, vorzugsweise solche mit 1-20 C-Atomen umgesetzt werden. Insbesondere sind die folgenden zu nennen:
Carbonsäuren, wie z.B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure, o-Chlorbenzoesäure, p-Chlorbenzoesäure, o-Nitrobenzoesäure, p-Nitrobenzoesäure, Salicylsäure, p-Hydroxybenzoesäure, Anthranilsäure, p-Aminobenzoesäure, Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Phthalsäure, Isophthalsäure, Terephthalsäure;
Carbonsäureester, wie z.B. die C₁-C₁₀-Alkylester der oben genannten Carbonsäuren, insbesondere Methylformiat, Essigester, Buttersäurebutylester, Phthalsäure-, Isophthalsäure-, Terephthalsäure-, Adipinsäure-, Maleinsäuredialkylester wie z.B. die Dimethylester dieser Säuren, (Meth)acrylsäuremethylester, Butyrolacton, Caprolacton und Polycarbonsäureester, wie z.B. Polyacryl- und Polymethacrylsäureester und deren Copolymere und Polyester, wie z.B. Polymethylmethacrylat, Terephthalsäureester und andere technische Kunststoffe, wobei hier insbesondere Hydrogenolysen, also die Umsetzung von Estern zu den entsprechenden Säuren und Alkoholen, durchgeführt werden;
Fette;
Carbonsäureanhydride, wie z.B. die Anhydride der oben genannten Carbonsäuren, insbesondere Essigsäureanhydrid, Propionsäureanhydrid, Benzoesäureanhydrid und Maleinsäureanhydrid;

Carbonsäureamide, wie z.B. Formamid, Acetamid, Propionamid, Stearamid, Terephthalsäureamid.

Ferner können auch Hydroxycarbonsäuren, wie z.B. Milch-, Äpfel-, Wein- oder Zitronensäure, oder Aminosäuren, wie z.B. Glycin, Alanin, Prolin und Arginin, und Peptide umgesetzt werden.

Als besonders bevorzugte organische Verbindungen werden gesättigte oder ungesättigte Carbonsäuren, Carbonsäureester, Carbonsäureanhydride oder Lactone oder Gemische aus zwei oder mehr davon hydriert.

Demgemäss betrifft die vorliegende Erfindung auch ein Verfahren, wie oben beschrieben, das dadurch gekennzeichnet ist, dass die organische Verbindung eine Carbonsäure, ein Carbonsäureester, ein Carbonsäureanhydrid oder ein Lacton ist.

Beispiele dieser Verbindungen sind unter anderem Maleinsäure, Maleinsäureanhydrid, Bernsteinsäure, Bernsteinsäureanhydrid, Adipinsäure, 6-Hydroxycapronsäure, 2-Cyclododecylpropionsäure, die Ester der vorgenannten Säuren wie z.B. Methyl-, Ethyl-, Propyl- oder Butylester. Weitere Beispiele sind γ-Butyrolacton und Caprolacton.

In einer ganz besonders bevorzugten Ausführungsform betrifft die vorliegende Erfindung ein Verfahren, wie oben beschrieben, das dadurch gekennzeichnet ist, dass die organische Verbindung Adipinsäure oder ein Adipinsäureester ist.

Die zu hydrierende Carbonylverbindung kann dem Hydrierungsreaktor allein oder als Gemisch mit dem Produkt der Hydrierungsreaktion zugeführt werden, wobei dies in unverdünnter Form oder unter Verwendung von zusätzlichem Lösungsmittel geschehen kann. Als zusätzliches Lösungsmittel eigenen sich insbesondere Wasser, Alkohole wie Methanol, Ethanol und der Alkohol, der unter den Reaktionsbedingungen entsteht. Bevorzugte Lösungsmittel sind Wasser, THF und NMP, besonders bevorzugt ist Wasser.
Die Hydrierung sowohl in Sumpf- als auch in Rieselfahrweise, wobei jeweils bevorzugt in Kreistauffahrweise gearbeitet wird, führt man im allgemeinen bei einer Temperatur im Bereich von 50 bis 350°C, bevorzugt im Bereich von 70 bis 300°C, besonders bevorzugt im Bereich von 100 bis 270°C und einem Druck im Bereich von 3 bis 350 bar, bevorzugt im Bereich von 5 bis 330 bar, besonders bevorzugt im Bereich von 10 bis 300 bar durch.

In einer ganz besonders bevorzugten Ausführungsform werden die Katalysatoren in Verfahren zur Herstellung von Hexandiol und/oder Caprolacton eingesetzt, wie sie in DE 196 07 954, DE 196 07 955, DE 196 47 348 und DE 196 47 349 beschrieben sind.

Mit dem erfindungsgemäßen Verfahren unter Verwendung der Katalysatoren werden hohe Umsätze und Selektivitäten erzielt. Gleichzeltig weisen die Katalysatoren eine hohe chemische und mechanische Stabilität auf.

Die mechanische Stabilität von Festkörperkatalysatoren und speziell der erfindungsgemäß verwendeten Katalysatoren wird beschrieben durch den Parameter Seitendruckfestigkeit in verschiedenen Zuständen (oxidisch, reduziert, reduziert und unter Wasser suspendiert).

Die Seitendruckfestigkeit wurde im Rahmen der vorliegenden Anmeldung bestimmt mit einem Gerät des Typs "Z 2.5/T 919" der Firma Zwick (Ulm). Sowohl bei den reduzierten als auch bei den gebrauchten Katalysatoren wurden die Messungen unter Stickstoffatmosphäre durchgeführt, um eine Re-Oxidation der Katalysatoren zu vermeiden.
In den folgende Beispielen soll die Erfindung näher beschrieben werden.

### Beispiele

### Beispiel 1: Herstellung des Katalysators 1

### Herstellung des Katalysators

Ein Gemisch aus 12,41 kg einer 19,34%igen-Kupfernitratlösung, und 14,78 kg einer 8,12%igen-Aluminiumnitratlösung und 1,06 kg einer 37,58%igen Lanthannitratlösung x 6H₂O wurden in 1,5 l Wasser gelöst (Lösung 1). Lösung 2 beinhaltet 60 kg einer 20 %igen-wasserfreies Na₂CO₃. Lösung 1 und Lösung 2 werden über getrennte Leitungen in ein Fällgefäß, das mit einem Rührer versehen ist und 10 l auf 60°C erhitztes Wasser enthält, geleitet. Hierbei wurde durch entsprechende Einstellung der Zufuhrgeschwindigkeiten der Lösung1 und Lösung 2 der pH-Wert auf 6,2 gebracht.

Unter Konstanthaltung des pH-Wertes bei 6,2 und der Temperatur bei 60°C wurde die gesamte Lösung1 mit Soda zur Reaktion gebracht. Die so gebildete Suspension wurde anschließend 1 Stunden lang nachgerührt, wobei der pH-Wert durch gelegentliche Zugabe von verdünnter Salpetersäure bzw. Sodalösung 2 auf bei 7,2 gefahren wird. Die Suspension wird filtriert und mit destilliertem Wasser so lange gewaschen, bis der Nitratgehalt des Waschwassers < 10 ppm betrug.

Der Filterkuchen wurde 16 h lang bei 120°C getrocknet und anschließend 2h lang bei 300°C calciniert. Das so erhaltene Katalysatorpulver wird mit 1 Gew.-% Graphit vorkompaktiert. Das erhaltene Kompaktat wird mit 5 Gew.% Cu-Blättchen Unicoat und anschließend mit 2 Gew.% Graphit gemischt und zu Tabletten von 3 mm Durchmesser und 3 mm Höhe verpresst. Die Tabletten wurden schließlich 2 h lang bei 350°C calciniert.

Der so hergestellte Katalysator hat die chemische Zusammensetzung 57% CuO /28,5 % Al₂O₃ / 9,5% La₂O₃/ 5 % Cu. Die Seitendruckfestigkeit im oxidischen und reduzierten Zustand sind in Tabelle 1 aufgeführt.

### Beispiel 2: Hydrierung von Adipinsäuredimethylester an Katalysator 1

Adipinsäuredimethylester wurde kontinuierlich in Rieselfahrweise mit Rückführung (Verhältnis Zulauf/Rückführung = 10/1) bei einer Belastung von 0,3 kg/(l*h), einem Druck von 200 bar und Reaktionstemperaturen von 220°C bzw. 240°C in einem senkrechten Rohrreaktor, der mit 200 ml Katalysator 1 gefüllt war, hydriert. Die Versuchsdauer betrug insgesamt 7 Tage. GC-analytisch wurden im Reaktoraustrag bei 220°C bzw. 240°C Esterumsätze von 98-99 % bzw. 99 %, Hexandiol-Anteile von 57 % bzw. 62 % detektiert. Nach Ausbau war der Katalysator noch voll erhalten und wies eine hohe mechanische Stabilität auf. Seitendruckfestigkeit sind in Tabelle 1 zusammengestellt. Die Versuchsergebnisse sind nochmals in Tabelle 2 zusammengefasst.

### Beispiel 3: Herstellung des Vergleichkatalysators

Der Vergleichskatalysator wurde analog dem Katalysator 2 hergestellt, jedoch ohne die Zugabe der Lanthannitratlösung, das bedeutet: 14,5 kg einer 19,34%gen Kupfernitratlösung und 14,5 kg einer 8,12%igen Aluminiumnitratlösung (Lösung 1) werden mit einer Sodalösung analog Katalysator 1 gefällt.

Der so hergestellte Katalysator hat die chemische Zusammensetzung 66,5 % CuO /28,5 % Al₂O₃ / 5 % Cu. Die Seitendruckfestigkeit im oxidischen und reduzierten Zustand sind in Tabelle 1 aufgeführt.

### Beispiel 4: Hydrierung von Adipinsäuredimethylester am Vergleichskatalysator

Adipinsäuredimethylester wurde kontinuierlich in Rieselfahrweise mit Rückführung (Verhältnis Zulauf / Rückführung = 10/1) bei einer Belastung von 0,3 kg/(l*h), einem Druck von 200 bar und Reaktionstemperaturen von 220°C bzw. 240°C in einem senkrechten Rohrreaktor, der mit 200 ml Katalysator 2 gefüllt war, hydriert. Die Versuchsdauer betrug insgesamt 7 Tage. GC-analytisch wurden im Reaktoraustrag bei 220°C bzw. 240°C Esterumsätze von jeweils 98 %, Hexandiol-Anteile von 55 % detektiert. Nach Ausbau war der Katalysator noch voll erhalten und wies eine hohe mechanische Stabilität auf. Seitendruckfestigkeiten sind in Tabelle 1 zusammengestellt. Die Versuchsergebnisse sind nochmals in Tabelle 2 zusammengefasst.

**Tabelle 1**

| Katalysator | Seiten-druckfestigkeit (oxidisch) / N | Seitendruckfestigkeit (reduziert) / N | Seitendruckfestigkeit (nach Ausbau) / N | Seitendruckfestigkeit (reduziert, unter Wasser suspendiert) / N |
|---|---|---|---|---|
| Katalysator 1 | 111 | 62 | 51 | 41 |
| Vergleichskatalysator | 70 | 45 | 20 | 26 |

Die Daten in Tabelle 1 zeigen, dass der erfindungsgemäß verwendete Katalysator 1 im reduzierten Zustand und nach Ausbau eine deutlich höhere mechanische Stabilität, zeigen als der Vergleichskatalysator.

Die Daten in der folgenden Tabelle 2 zeigen, dass die erfindungsgemäß verwendeten Katalysatoren signifikant höhere Hydrieraktivitäten, d.h. höhere Umsätze an Adipinsäuredimethylester bei 220°C bzw. 240°C aufweisen als der Vergleichskatalysator, sowie auch tendenziell höherer Wertproduktselektivitäten, d.h. Gehalte an den Zielprodukten Hexandiol im Austrag.

**Tabelle 2**

| Katalysator | Reaktionstemperatur /°C | Umatz Adipinsäuredimethylester / % | Hexandiolgehalt im Austrag / % |
|---|---|---|---|
| Katalysator 1 | 220 | 98 | 57 |
| | 240 | 99 | 62 |
| Vergleichskatalysator | 220 | 92 | 48 |
| | 240 | 96 | 58 |

## Patentansprüche

1. Verfahren zur Hydrierung einer mindestens eine Carbonylgruppe aufweisenden organischen Verbindung, bei dem die organische Verbindung in Anwesenheit von Wasserstoff mit einem Formkörper in Kontakt gebracht wird, der herstellbar ist gemäß einem Verfahren, in dem
(i) ein oxidisches Material, umfassend Kupferoxid, Aluminiumoxid und Lanthanoxid, bereitgestellt wird,
(ii) dem oxidischen Material pulverförmiges metallisches Kupfer, oder Kupferblättchen zugegeben wird, und
(iii) das aus (ii) resultierende Gemisch zu einem Formkörper verformt wird.

2. Verfahren nach Anspruch 1, **dadurch gekennzeichnet, dass** dem oxidischen Material oder dem aus (ii) resultierendem Gemisch Graphit in einem Anteil im Bereich von 0,5 bis 5 Gew.-%, bezogen auf das Gesamtgewicht des oxidischen Materials, zugegeben wird.

3. Verfahren nach Anspruch 1 oder 2, **dadurch gekennzeichnet, dass** die organische Verbindung eine Carbonsäure, ein Carbonsäureester, ein Carbonsäureanhydrid oder ein Lacton ist.

4. Verfahren nach Anspruch 3, **dadurch gekennzeichnet, dass** die organische Verbindung Adipinsäure oder ein Adipinsäureester ist.

## Claims

1. A process for hydrogenating an organic compound which has at least one carbonyl group, in which the organic compound is brought into contact in the presence of hydrogen with a shaped article which can be produced in a process in which
(i) an oxidic material comprising copper oxide, aluminum oxide and lanthanum oxide is prepared,
(ii) powdered metallic copper, copper flakes, powdered cement, graphite or a mixture thereof is added to the oxidic material, and
(iii) the mixture resulting from (ii) is shaped to a shaped article.

2. The process according to claim 1, wherein graphite is added in a content in the range from 0.5 to 5% by weight based on the total weight of the oxidic material to the oxidic material or to the mixture resulting from (ii).

3. The process according to claim 1 or 2, wherein the organic compound is a carboxylic acid, a carboxylic ester, a carboxylic anhydride or a lactone.

4. The process according to claim 3, wherein the organic compound is adipic acid or an adipic ester.

## Revendications

1. Procédé d'hydrogénation d'un composé organique comportant au moins un groupe carbonyle, selon lequel le composé organique est mis en contact en présence d'hydrogène avec un corps moulé, qui peut être fabriqué par un procédé, selon lequel
(i) un matériau oxydique, comprenant de l'oxyde de cuivre, de l'oxyde d'aluminium et de l'oxyde de lanthane, est mis à disposition,
(ii) du cuivre métallique pulvérulent, des lamelles de cuivre, du ciment pulvérulent, du graphite ou un de leurs mélanges est ajouté au matériau oxydique, et
(iii) le mélange résultant de (ii) est façonné en un corps moulé.

2. Procédé selon la revendication 1, **caractérisé en ce que** du graphite est ajouté au matériau oxydique ou au mélange résultant de (ii) en une proportion dans la plage allant de 0,5 à 5 % en poids, par rapport au poids total du matériau oxydique.

3. Procédé selon la revendication 1 ou 2, **caractérisé en ce que** le composé organique est un acide carboxylique, un ester d'acide carboxylique, un anhydride d'acide carboxylique ou une lactone.

4. Procédé selon la revendication 3, **caractérisé en ce que** le composé organique est l'acide adipique ou un ester de l'acide adipique.
